(19) Europäisches Patentamt
European Patent Office
Office européen des brevets

(11) **EP 4 603 874 A1**

(12) **EUROPEAN PATENT APPLICATION**
published in accordance with Art. 153(4) EPC

(43) Date of publication:
**20.08.2025 Bulletin 2025/34**

(21) Application number: **23896886.1**

(22) Date of filing: **30.11.2023**

(51) International Patent Classification (IPC):
**G01S 15/89** (2006.01) **A61B 8/00** (2006.01)

(52) Cooperative Patent Classification (CPC):
**A61B 8/00; G01S 15/89**

(86) International application number:
**PCT/CN2023/135521**

(87) International publication number:
**WO 2024/114744 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2022 CN 202211513999**

(71) Applicant: **Wuhan United Imaging Healthcare Co., Ltd.**
**WuHan, Hubei 430206 (CN)**

(72) Inventors:
• **ZHANG, Jianmin**
**Wuhan, Hubei 430206 (CN)**
• **SHI, Aiwei**
**Wuhan, Hubei 430206 (CN)**

(74) Representative: **Wang, Bo**
**Panovision IP**
**Ebersberger Straße 3**
**85570 Markt Schwaben (DE)**

(54) **ULTRASOUND IMAGING METHOD, SYSTEM AND APPARATUS, AND STORAGE MEDIUM**

(57)    Provided in the embodiments of the present description are an ultrasound imaging method, system and apparatus, and a storage medium, The method is executed by a processor, and comprises: determining a delay time of each pixel point in an active area on the basis of an echo signal of a focusing wave, determining from the active area a plurality of reference pixel points and delay times corresponding to the plurality of reference pixel points, determining a delay time of a target pixel point in an artifact area on the basis of the plurality of reference pixel points and the delay times corresponding to the plurality of reference pixel points. wherein the target pixel point is located in an area defined by the plurality of reference pixel points, and generating an ultrasound image on the basis of the delay time of each pixel point in the active area and the delay time of the target pixel point in the artifact area.

200

Determining a delay time of each pixel point in an effective region based on an echo signal of a focused wave — 210

Determining reference pixel points from the effective region and delay times corresponding to the reference pixel points — 220

Determining a delay time of each of one or more target pixel points in an artifact region based on the reference pixel points and the delay times corresponding to the reference pixel points — 230

Generating an ultrasonic image based on the delay time of each pixel point in the effective region and the delay time of each of the one or more target pixel points in the artifact region — 240

**FIG. 2**

**Description**

**CROSS-REFERENCE TO RELATED APPLICATIONS**

This application claims priority to Chinese Patent Application No. 202211513999.0, filed on November 30, 2022, the entire content of which is hereby incorporated by reference.

**TECHNICAL FIELD**

The present disclosure relates to the field of ultrasonic imaging, and in particular relates to ultrasonic imaging method, device, computer device and storage medium.

**BACKGROUND**

Beamforming is a crucial part of an ultrasonic imaging chain. Currently, emission focusing for ultrasonic imaging may be achieved through a point source model. However, an acoustic wave propagation model near a focal point is complex, and the use of the point source model in ultrasonic imaging often results in degraded image quality near the focal point, forming a dark zone. Therefore, how to calculate beamforming for an artifact region not covered by the point source model and improve image quality is an urgent problem to be solved in the field.

In view of this, CN103837608B discloses a method and a system for phased array reception dynamic focusing compensation. The system receives echo data, inserts initial focusing delays into the echo data based on a difference in propagation times, initiates delay compensation on the echo data, and finally superimposes echo data dynamically focused and compensated. The system can dynamically compensate for the delays of echo data focused at different depths, reducing imaging distortion and improving image quality at various depths. The system also meets focusing compensation requirements for all depths under refraction conditions. However, the system requires delay compensation for each echo data, resulting in a large computational load, and it is difficult to adapt to different needs.

Therefore, it is desirable to provide a method, an apparatus, computer device, and storage medium for ultrasonic imaging that solve the problem of beamforming calculation for an artifact region not covered by the point source model by ensuring smooth transitions in the calculated transmission delays, thereby effectively eliminates a dark zone (artifacts) near a focal point and improves imaging quality.

**SUMMARY**

One or more embodiments of the present disclosure provide a method for ultrasonic imaging. The method includes: determining a delay time of each pixel point in an effective region based on an echo signal of a focused wave, determining reference pixel points from the effective region and delay times corresponding to the reference pixel points, and determining a delay time of each of one or more target pixel points in an artifact region based on the reference pixel points and the delay times corresponding to the reference pixel points. The one or more target pixel points are located within a region enclosed by the reference pixel points. The method further includes: generating an ultrasonic image based on the delay time of each pixel point in the effective region and the delay time of each of the one or more target pixel points in the artifact region.

One or more embodiments of the present disclosure provide a system for ultrasonic imaging. The system includes a first determination module, a second determination module, a third determination module, and an image generation module. The first determination module is configured to determine a delay time of each pixel point in an effective region based on an echo signal of a focused wave. The second determination module is configured to determine reference pixel points from the effective region and delay times corresponding to the reference pixel points. The third determination module is configured to determine a delay time of each of one or more target pixel points in an artifact region based on the reference pixel points and the delay times corresponding to the reference pixel points, wherein the one or more target pixel points are located within a region enclosed by the reference pixel points. The image generation module is configured to generate an ultrasonic image based on the delay time of each pixel point in the effective region and the delay time of each of the one or more target pixel points in the artifact region.

One or more embodiments of the present disclosure provide a device for ultrasonic imaging. The device includes at least one processor and at least one storage device. The at least one storage device is configured to store computer instructions. The at least one processor is configured to execute at least a portion of the computer instructions to implement the method for ultrasonic imaging described in any of the above embodiments of the present disclosure.

One or more embodiments of the present disclosure provide a non-transitory computer-readable storage medium storing computer instructions. When a computer reads the computer instructions from the storage medium, the computer executes the method for ultrasonic imaging described in any of the above embodiments of the present disclosure.

## BRIEF DESCRIPTION OF THE DRAWINGS

The present disclosure will be further illustrated by way of exemplary embodiments, which will be described in detail through the accompanying drawings. These embodiments are not limiting, and in these embodiments, the same numbering denotes the same structure, wherein:

FIG. 1 is an exemplary block diagram of an ultrasonic imaging system according to some embodiments of the present disclosure;
FIG. 2 is a flowchart of an exemplary process for ultrasonic imaging according to some embodiments of the present disclosure;
FIG. 3 is a flowchart of an exemplary process for determining a delay time of each of one or more target pixel points according to some embodiments of the present disclosure;
FIG. 4 is a flowchart of another exemplary process for determining a delay time of each of one or more target pixel points according to some embodiments of the present disclosure;
FIG. 5 is a flowchart of an exemplary process for determining a delay time of each of one or more intermediate pixel points according to some embodiments of the present disclosure;
FIG. 6 is a flowchart of an exemplary process for determining a delay time of each of one or more target pixel points according to some embodiments of the present disclosure;
FIG. 7A is a schematic diagram of an effective region and an artifact region according to some embodiments of the present disclosure;
FIG. 7B is a schematic diagram of a target pixel point located within a triangular region formed by three reference pixel points according to some embodiments of the present disclosure;
FIG. 7C is a schematic diagram illustrating a target pixel point located within a quadrilateral region formed by four reference pixel points according to some embodiments of the present disclosure;
FIG. 8A is a schematic diagram of a target overlap point and a target region according to some embodiments of the present disclosure;
FIG. 8B is a schematic diagram illustrating overlapping between artifact regions derived from adjacent focused wave emissions according to some embodiments of the present disclosure;
FIG. 8C is a schematic diagram illustrating overlapping between artifact regions derived from adjacent focused wave emissions according to some other embodiments of the present disclosure;
FIG. 9A is a schematic diagram of a first area and a second area according to some embodiments of the present disclosure; and
FIG. 9B is a schematic diagram illustrating the determination an intermediate pixel point according to some embodiments of the present disclosure.

## DETAILED DESCRIPTION

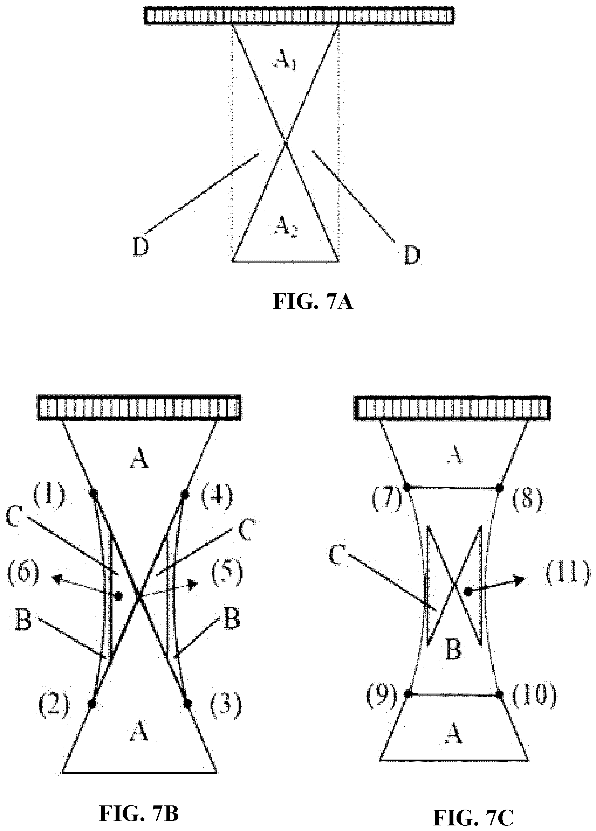

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. Unless otherwise indicated or stated in the context, the same reference numerals in the drawings represent the same structures or operations.
It should be understood that the terms "system," "device," "unit," and/or "module" used herein are ways for distinguishing different levels of components, elements, parts, or assemblies. However, if other terms can achieve the same purpose, they may be used as alternatives.
As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.
Flowcharts are used in the present disclosure to illustrate the operations performed by the system according to the embodiments described herein. It should be understood that the operations may not necessarily be performed in the exact sequence depicted. Instead, the operations may be performed in reverse order or concurrently. Additionally, other operations may be added to these processes, or one or more operations may be removed.
Taking an ultrasonic imaging system that includes an array element and a Field Programmable Gate Array (FPGA) as an example, in a point source model, an emission aperture of the array element is moved to emit an ultrasonic wave toward the object to be imaged and receive an echo signal from the object. The FPGA then calculates a delay time of each pixel point based on a spatial position of the object, thereby reconstructing an ultrasonic image of the object based on the delay time of the each pixel point. Referring to FIG. 7A, the array element includes multiple channels, and a portion of the multiple channels in the array element is selected as the emission aperture. When emitting ultrasonic waves, channels located at two sides of the emission aperture typically emit ultrasonic waves first, while channels in a middle region of the emission aperture emit ultrasonic waves later, ensuring that the ultrasonic waves emitted by the emission aperture forms a focused wave.

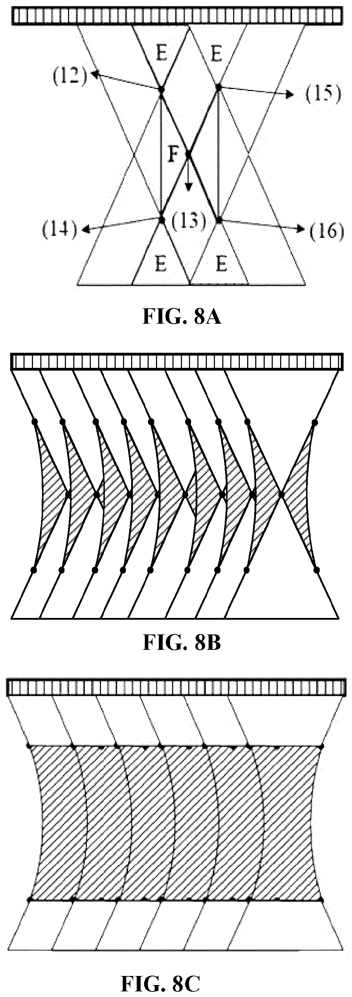

Furthermore, each channel on the array element may receive an echo signal. The FPGA may calculate an emission delay and a reception delay for each pixel point based on the echo signal. Based on the delay time of each pixel point, the corresponding echo signal at each moment is determined, and an imaging region of an ultrasonic image is reconstructed, as shown by the two triangular regions $A_1$ and $A_2$ in FIG. 7A. At the same time, as shown in FIG. 8A, in the point source model, regardless of how the emission aperture moves, the imaging region determined by the emission delay and the reception delay always narrows near a focal point, resulting in the issue of dark zone artifacts near a focal region in ultrasonic imaging.

In view of the above, it is necessary to address the aforementioned technical problem by providing an ultrasonic imaging method that can improve the imaging quality near the focal region. Some embodiments of the present disclosure provide an ultrasonic imaging method that enhances imaging quality. By using the delay time of the focal point within an effective imaging region, the delay times of pixel points in the region near the focal point (also referred to as a dark zone) are indirectly determined. The method achieves smooth transitions at hard boundaries, solves the problem of difficult delay calculations near the focal point in point source model imaging, effectively eliminates imaging artifacts, and improves imaging quality.

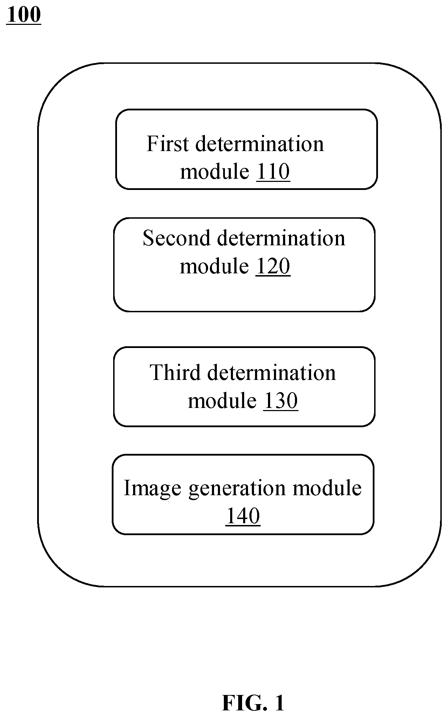

FIG. 1 is an exemplary block diagram of an ultrasonic imaging system according to some embodiments of the present disclosure. As shown in FIG. 1, an ultrasonic imaging system 100 (hereinafter referred to as the system 100) may include a first determination module 110, a second determination module 120, a third determination module 130, and an image generation module 140. The following will provide a detailed description of the system 100 involved in the embodiments of the present disclosure. It should be noted that the following embodiments are only used to explain the present disclosure and do not limit the scope of the present disclosure.

In some embodiments, the first determination module 110 may be configured to determine a delay time of each pixel point in an effective region based on an echo signal of a focused wave. In some embodiments, the first determination module 110 may further be configured to determine the delay time of the each pixel point in the effective region by processing the echo signal of the focused wave using a point source model.

In some embodiments, the second determination module 120 may be configured to determine reference pixel points from the effective region and determine delay times corresponding to the reference pixel points.

In some embodiments, the third determination module 130 may be configured to determine a delay time of each of one or more target pixel points in an artifact region based on the reference pixel points and the delay times corresponding to the reference pixel points, and the one or more target pixel points are located within a region enclosed by the reference pixel points.

In some embodiments, the third determination module 130 may be further configured to: for each of the one or more target pixel points, determine a delay time of the target pixel point based on delay times of the reference pixel points, position information of the reference pixel points, and position information of the target pixel point.

In some embodiments, the third determination module 130 may be further configured to: determine geometric information between the target pixel point and the reference pixel points based on the position information of the reference pixel points and the position information of the target pixel point; and determine the delay time of the target pixel point based on the geometric information and the position information of the reference pixel points.

In some embodiments, when the target pixel point is located within a triangular region enclosed by three reference pixel points, the geometric information includes area information of a region formed by the target pixel point and the three reference pixel points, the third determination module 130 may be further configured to: determine three first areas based on position information of any two of the three reference pixel points and the position information of the target pixel point; determine a second area based on the position information of the three reference pixel points; and determine the delay time of the target pixel point based on the three first areas, the second area, and the delay times of the three reference pixel points.

In some embodiments, the third determination module 130 may be further configured to: for each first area of the three first areas, determine a ratio of the each first area to the second area; determine a product of the ratio corresponding to the each first area and the delay time of the reference pixel point corresponding to the each first area; and determine the delay time of the target pixel point based on products corresponding to the three first areas.

In some embodiments, the effective region is composed of a first effective region and a second effective region formed by a propagation of the focused wave. When the target pixel point is located within a quadrilateral region enclosed by four reference pixel points, the geometric information includes line segment information of a region formed by the target pixel point and the four reference pixel points, the third determination module 130 may be further configured to determine a first line segment and one or more second line segments based on position information of the four reference pixel points and the position information of the target pixel point. The first line segment is a line segment that passes through the target pixel point and intersects with the one or more second line segments, and each of the one or more second line segments connects a reference pixel point in the first effective region with a reference pixel point in the second effective region. The third determination module 130 may be further configured to: determine position information of one or more intermediate pixel points. Each of the one or more intermediate pixel points is an intersection point of the first line segment and one of the one or more second line segments. The third determination module 130 may be further configured to determine a delay time of each of the one or more intermediate pixel points based on the position information of the intermediate pixel point and position information and delay times of reference pixel points at two ends of the second line segment where the intermediate pixel point is located, and determine the delay time of the target pixel point based on the delay times of the one or more intermediate pixel points

In some embodiments, the third determination module 130 may be further configured to: determine first distances and a second distance based on the position information of the intermediate pixel point and the position information of the reference pixel points at the two ends of each of the one or more second line segments where the intermediate pixel point is located. Each of the first distances is a distance between the intermediate pixel point and a reference pixel point at an end of the second line segment where the intermediate pixel point is located, and the second distance is a distance between the reference pixel points at the two ends of the second line segment. The third determination module 130 may be further configured to determine, for each of the first distances, a first distance ratio of the first distance to the second distance, determine a first distance product result of the delay time of the reference pixel point corresponding to the first distance and the first distance ratio, and determine the delay time of the intermediate pixel point based on first distance product results corresponding to the first distances.

In some embodiments, the third determination module 130 may be further configured to: determine third distances and a fourth distance based on the position information of the intermediate pixel point and the position information of the target pixel point. Each of the third distances is a distance between the target pixel point and an intermediate pixel point at an end of the first line segment, and the fourth distance is a distance between intermediate pixel points at two ends of the first line segment. The third determination module 130 may be further configured to determine, for each of the third distances, a second distance ratio of the third distance to the fourth distance, determine a second distance product result of the delay time of the intermediate pixel point corresponding to the third distance and the second distance ratio corresponding to the third distance, and determine the delay time of the target pixel point based on second distance product results corresponding to the third distances.

In some embodiments, the image generation module 140 may be configured to generate an ultrasonic image based on the delay time of each pixel point in the effective region and the delay time of each of the one or more target pixel points in the artifact region.

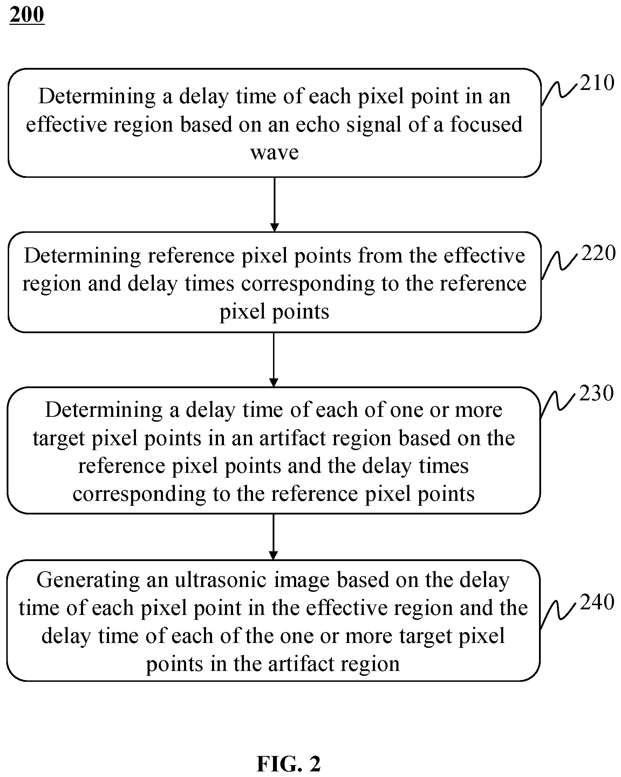

More descriptions of the effective region, the artifact region, the delay time, etc., may be found in related descriptions in the present disclosure (e.g., FIG. 2 and the related descriptions thereof).

It should be noted that the above description of the system 100 and the modules thereof is for convenience of explanation only and should not be construed as limiting the scope of the present disclosure to the described embodiments. It may be understood that for those skilled in the field, after understanding the principles of the system, they may combine the modules in any manner or form subsystems connected with other modules without departing from these principles. In some embodiments, the first determination module 110, the second determination module 120, the third determination module 130, and the image generation module 140 disclosed in FIG. 1 may be different modules within a system, or a single module may implement the functions of two or more of the aforementioned modules. For example, the modules may share a common storage module, or each module may have its own storage module. Such variations fall within the scope of protection of the present disclosure.

The ultrasonic imaging system 100 can be applied to an ultrasonic imaging device. In some embodiments, the ultrasonic imaging device may be a server. The ultrasonic imaging device includes a processor, a storage device, and a network interface connected via a system bus. The processor of the ultrasonic imaging device is configured to provide computing and control capabilities. The storage device of the ultrasonic imaging device includes a non-volatile storage medium and an internal memory. The non-volatile storage medium stores an operating system, computer instructions, and a database. The internal memory provides an environment for the execution of the operating system and computer instructions in the non-volatile storage medium. The database of the ultrasonic imaging device is configured to store relevant data. The network interface of the ultrasonic imaging device is configured to communicate with external terminals via a network connection. The computer instructions, when executed by the processor, implement an ultrasonic imaging method. In some embodiments, the ultrasonic imaging device may include more or fewer components, combine certain components, or have a different arrangement of components.

Specifically, the ultrasonic imaging device may be a Central Processing Unit (CPU), a Digital Signal Processor (DSP), a Field-Programmable Gate Array (FPGA), or other programmable logic devices. This embodiment does not impose any limitations in this regard.

FIG. 2 is a flowchart of an exemplary process for ultrasonic imaging according to some embodiments of the present disclosure. As shown in FIG. 2, process 200 may include the following operations. In some embodiments, one or more operations of the process 200 illustrated in FIG. 2 may be implemented in an ultrasonic imaging device. For example, process 200 shown in FIG. 2 may be stored in the form of instructions in a storage device and invoked and/or executed by a processor.

In 210, the processor acquires an echo signal of a focused wave and determines a delay time of each pixel point in an effective region.

The focused wave refers to an ultrasonic wave that undergoes focusing during emission. An ultrasonic probe is equipped with an array element (e.g., the long rectangular portion at the top of FIG. 7A), and the array element includes multiple channels (e.g., the small rectangular portions within the long rectangle at the top of FIG. 7A). A portion of the channels of the array element is selected as an emission aperture (also referred to as an emission element) for emitting ultrasonic waves. At the same time, each channel of the array element may also act as a receiving channel (also referred to as a receiving element) for receiving an echo signal. The processor may control the emission element to emit ultrasonic waves in a continuous manner. For example, the processor may first control a 1st array element to a 20th array element of the ultrasonic probe to emit ultrasonic waves, then control a 5th array element to a 25th array element to emit ultrasonic waves, and so on.

The echo signal refers to a wave signal that is reflected back from a reflector over a continuous time period. In some embodiments, the echo signal of the focused wave may include an amplitude, a frequency, etc., of the focused wave. In some embodiments, multiple sets of echo signals of focused waves required for imaging are acquired by moving the emission aperture and the focal position.

The effective region refers to an effective emission region of the focused wave, i.e., a propagation region of the focused wave. For example, portions $A_1$ and $A_2$ (the two triangles) shown in FIG. 7A represent the effective regions of a single focused wave emission.

In some embodiments, the effective region may be determined based on a propagation path of the focused wave, and the effective region is composed of a first effective region and a second effective region formed by a propagation of the focused wave.

The propagation path refers to a route of each emission and reception of the focused wave.

The first effective region refers to a region formed by the propagation paths of the focused wave from emission to a focal point, for example, the region $A_1$ (i.e., the upper triangle) in FIG. 7A.

The second effective region refers to a region formed by the propagation paths of the focused wave after the focused wave leaves the focal point. For example, after passing through the focal point, the focused wave continues to propagate in a diverging manner, forming the region $A_2$ (i.e., the lower triangle) in FIG. 7A.

The delay time may be a sum of an emission delay and a reception delay. Alternatively, the delay time may be the emission delay or the reception delay. The emission delay refers to a duration from a time when an emission channel of the emission aperture emits an ultrasonic wave to a time when the ultrasonic wave reaches the focal point. The reception delay refers to a duration from a time when the ultrasonic wave reaches the focal point to a time when the ultrasonic wave is received by a receiving channel of the array element. In some embodiments, the processor may determine the delay time of a pixel point based on a distance from the emission aperture to the pixel point and a distance from the pixel point to the receiving channel. Since the distances from different channels to a same position are different, the delay times from the pixel point to different channels need to be calculated.

In some embodiments, the delay time of a pixel point in the effective region may be a sum of the emission delay and the reception delay of the pixel point, or the delay time of a pixel point may be the emission delay of the pixel point.

In some embodiments, the processor may determine the delay time of each pixel point in the effective region based on the echo signal of the focused wave, using the delay calculation principle of focused wave imaging and a point source model. The point source model refers to a model constructed for processing and imaging an acoustic wave based on a propagation mode of the acoustic wave. The propagation mode of the acoustic wave may include a secondary propagation of the acoustic wave centered on the focal point when the acoustic wave reaches the focal point of emission. In the present disclosure, the echo signal of the focused wave may be analyzed and processed based on the point source model to eliminate a dark zone near the focal point and improve the quality of ultrasonic imaging. The focal point of each emission may be regarded as a point source. For example, if the effective region includes pixel points 1 to 100, the processor may process the echo signal of the focused wave based on the point source model and the delay calculation principle of focused wave imaging to determine delay times 1 to 100 corresponding to the pixel points 1 to 100.

In 220, the processor determines reference pixel points from the effective region and delay times corresponding to the reference pixel points.

It may be understood that, to ensure the completeness of ultrasonic imaging, the processor needs to supplement a delay time of each of one or more target pixel points in an artifact region. The artifact region refers to a region other than the effective region within an imaging region corresponding to the focused wave. The imaging region refers to all covered regions under the emission element corresponding to a focused imaging. As shown in FIG. 7A, the imaging region of an emission consists of regions $A_1$, $A_2$, and D, where regions $A_1$ and $A_2$ are the effective regions, and region D is the artifact region.

A target pixel point refers to a pixel point in the focused imaging for which the delay time needs to be supplemented. In some embodiments, the processor may determine all pixel points in the artifact region corresponding to an emission as target pixel points.

As shown in FIGs. 8A to 8C, when multiple emissions are performed in an ultrasonic imaging, the imaging regions corresponding to the multiple emissions may overlap. When the imaging regions corresponding to the multiple emissions overlap, for each emission, a portion of the artifact region corresponding to the emission overlaps with an effective region of an adjacent emission. Therefore, the delay time corresponding to the portion of the artifact region of the current emission may be obtained based on the effective region of the adjacent emission in ultrasonic imaging. For example, if an ultrasonic imaging includes three emissions as shown in FIG. 8A, an artifact region of a second emission from the left overlaps with an effective region of a first emission and an effective region of a third emission from the left. Therefore, the effective regions of the first emission and third emission from the left may supplement the delay times of the pixel points in an overlap region of the artifact region of the second emission from the left. However, since focused wave imaging narrows near a focal region, the effective regions of adjacent emissions may not completely cover all the artifact region corresponding to the current emission. To eliminate the dark zone near the focal point of focused imaging and ensure the quality of ultrasonic imaging, the one or more target pixel points at least include the pixel points in the artifact region corresponding to the current emission that are not covered by the effective regions of adjacent emissions.

In some embodiments, for each emission, the processor may determine one or more target overlap points between an effective region of the each emission and an effective region of an adjacent emission; determine a target region based on the focal pixel point and the one or more target overlap points; and determine the one or more target pixel points in the artifact region based on the target region. The one or more target pixel points in the artifact region at least include all pixel points within the target region.

A target overlap point is within an overlap region between the effective region of the each emission and the effective region of the adjacent emission and has a smallest distance to a focal pixel point of the each emission. The focal pixel point refers to a pixel point at a position of the focal point. As shown in FIG. 8A, regions E are the overlap regions between the effective region of the second emission and the effective regions of the adjacent first emission and third emission. Point (13) is the focal pixel point of the second emission, and points (12), (14), (15), and (16) are target overlap points.

In some embodiments, the processor may determine distances between all pixel points in the overlap region and the focal pixel point of the current emission and select a point with a smallest distance as a target overlap point of the current emission.

The target region refers to a region formed by the focal pixel point and the one or more target overlap points.

In some embodiments, for each emission, the processor may determine one or more target overlap points of the each emission and define the region formed by the one or more target overlap points and the focal pixel point of the each emission as the target region. For example, as shown in FIG. 8A, for the second emission, the region formed by points (12), (13), and (14) and the region formed by points (13), (15), and (16) may be the target region corresponding to that emission.

In some embodiments, the processor may determine the one or more target pixel points in the artifact region based on the target region. The one or more target pixel points in the artifact region may include all pixel points within the target region. If the one or more target pixel points in the artifact region include all pixel points within the target region, the delay times of the dark zone between emissions can be supplemented, thereby reducing artifacts in ultrasonic imaging.

In addition to all pixel points within the target region, the one or more target pixel points may also include other pixel points in the artifact region outside the target region to ensure overlap between target pixel points in artifact regions corresponding to multiple emissions, thereby avoiding significant abrupt changes in the imaging of the artifact regions corresponding to the multiple emissions. As shown in FIGs. 8B and 8C, the target pixel points may be pixels in regions indicated by diagonal lines, thereby ensuring overlap between the target pixel points corresponding to adjacent emissions and avoiding significant abrupt changes in the imaging of the artifact regions corresponding to the multiple emissions. In some embodiments, for each emission, there is a 30% to 80% overlap between one or more target pixel points corresponding to the each emission and one or more target pixel points corresponding to the adjacent emission. By limiting an overlap rate between the target pixel points of different emissions, a computational load on the processor is reduced, thereby improving computational efficiency and avoiding significant abrupt changes in the imaging of the artifact regions corresponding to multiple emissions.

In some embodiments of the present disclosure, by determining the one or more target overlap points and the target region, and further accurately determining the one or more target pixel points in the artifact region based on the target region, it is ensured that the selected target pixel point(s) can cover the dark zone between adjacent emissions, which is beneficial for subsequently improving the quality of focused wave imaging.

The reference pixel points refer to pixel points in the effective region used to supplement the delay times of the one or more target pixel points in the artifact region. The region formed by the reference pixel points must cover the one or more target pixel points in the artifact region, so that the processor can determine the delay times of the one or more target pixel points based on the reference pixel points. More descriptions of the determination of the delay times of the one or more target pixel points based on the reference pixel points may be found in the following sections of the present disclosure.

For example, if the one or more target pixel points include the pixel points in regions C shown in FIG. 7B, the processor may determine points (1), (2), (3), (4), and (5) as reference pixel points. The reference points (1), (2), (3), (4), and (5) may form two triangular-like regions, i.e., the two regions B shown in FIG. 7B. The two regions B may cover regions C, allowing the processor to further determine the delay time of each target pixel point (e.g., target pixel point (6) shown in FIG. 7B) in regions C based on points (1), (2), (3), (4), and (5).

As another example, if the one or more target pixel points include the pixel points in region C shown in FIG. 7C, the processor may determine points (7), (8), (9), and (10) as reference pixel points. The reference points (7), (8), (9), and (10) may form a rectangular-like region, i.e., region B shown in FIG. 7C. The region B may cover region C, allowing the processor to further determine the delay time of each target pixel point in region C based on points (7), (8), (9), and (10).

In some embodiments, the processor may determine position information of the one or more target pixel points in the artifact region and select reference pixel points from the pixel points in the effective region based on the position information, so that the region formed by the reference pixel points covers the one or more target pixel points in the artifact region. For example, the processor may determine a region formed by the one or more target pixel points and define the pixel points in the effective region closest to the vertices of the region as the reference pixel points. As another example, the processor may send the position information of the one or more target pixel points in the artifact region to a user (e.g., an operator of an ultrasonic imaging device), allowing the user to specify the reference pixel points.

It should be understood that, when the one or more target pixel points in the artifact region are determined, the reference pixel points are variable, as long as the region enclosed by the reference pixel points is sufficient to cover the one or more target pixel points. For example, the region enclosed by the reference pixel points may just cover the one or more target pixel points, or the region enclosed by the reference pixel points may be larger than a region where the one or more target pixel points are located.

In some embodiments, the processor may select pixel points near an edge of the effective region as the reference pixel points to reduce computational load and improve the efficiency of determining the delay times of the one or more target pixel points.

A reference delay time refers to a delay time corresponding to a reference pixel point. The processor may determine the reference pixel points and reference delay times corresponding to the reference pixel points based on the delay calculation principle of focused wave imaging.

In 230, the processor determines the delay time of each of the one or more target pixel points in the artifact region based on the reference pixel points and the reference delay times corresponding to the reference pixel points.

It should be understood that, for focused wave imaging, the processor may directly process the echo signal based on the point source model to determine the delay time of each pixel point in the effective region. However, during beamforming, the effective region narrows near the focal point, and there is no overlap between adjacent emissions, resulting in dark zones or artifacts in the artifact region (e.g., the diamond-shaped region in the middle as shown in FIG. 8A) near the focal point in ultrasonic imaging. This makes it impossible to directly determine the delay time of each pixel point in the artifact region.

Additionally, the imaging quality of the effective region is higher than the imaging quality of the artifact region. The imaging quality includes at least one of image signal uniformity or spatial resolution. For example, the image signal uniformity of the effective region is higher than the image signal uniformity of the artifact region, and the spatial resolution of the effective region is greater than the spatial resolution of the artifact region. Therefore, the processor may indirectly determine the delay times of the one or more target pixel points in the artifact region by using the reference pixel points from the effective region, thereby expanding an area of the artifact region and avoiding the issue of dark zones.

In some embodiments, the processor may determine the delay times of the one or more target pixel points in the artifact region by modeling or using various data analysis algorithms, such as regression analysis, discriminant analysis, etc., based on the reference pixel points and their corresponding reference delay times in the effective region. For example, the processor may input multiple reference pixel points into a trained computational model, and determine the delay time of each of the one or more target pixel points in the artifact region.

In some embodiments, for each of the one or more target pixel points, the processor may determine the delay time of the target pixel point in the artifact region based on the reference delay times, position information of the reference pixel points, and position information of the target pixel point. For example, the processor may perform modeling or use various data analysis algorithms, such as regression analysis, discriminant analysis, etc., to analyze and process the reference delay times, the position information of the reference pixel points, and the position information of the target pixel point, thereby determining the delay time of the target pixel point in the artifact region.

The position information of a reference pixel point refers to data information reflecting a position of the reference pixel point.

The position information of a target pixel point refers to data information reflecting a position of the target pixel point.

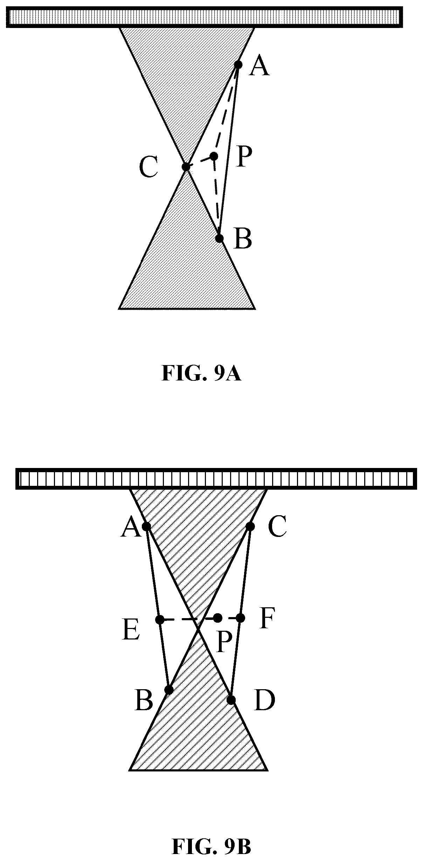

In some embodiments, the processor may obtain the position information of the reference pixel points and the position information of the target pixel point in various ways. For example, the processor may determine a position of the ultrasonic probe in a coordinate system of the imaging device and, based on a positional relationship between the ultrasonic probe and the reference pixel points as well as a positional relationship between the ultrasonic probe and the one or more target pixel points, determine the position information of the reference pixel points and the position information of the one or more target pixel points in the coordinate system of the imaging device. As another example, the processor may determine the position information of the reference pixel points and the position information of the one or more target pixel points based on preset position information of the array element and a speed of the ultrasonic wave. The position information may be represented by coordinates, such as the position information of point A in FIG. 9, which may be represented as $(x_1, y_1)$.

In some embodiments, for each of the one or more target pixel points, the processor may determine geometric information between the target pixel point and the reference pixel points based on the position information of the reference pixel points and the position information of the target pixel point. Then, based on the geometric information and the position information of the reference pixel points, the processor may determine the delay time of the target pixel point. The aforementioned geometric information may include information related to the positions of the target pixel point and the reference pixel points in the coordinate system of the imaging device. For example, the geometric information may include area information of a region formed by the target pixel point and the reference pixel points. As another example, the geometric information may include line segment information of the region formed by the target pixel point and the reference pixel points. More descriptions of the area information, the line segment information, and the determination of the delay time of the target pixel point based on the geometric information and the position information of the reference pixel points may be found in relevant sections below in the present disclosure.

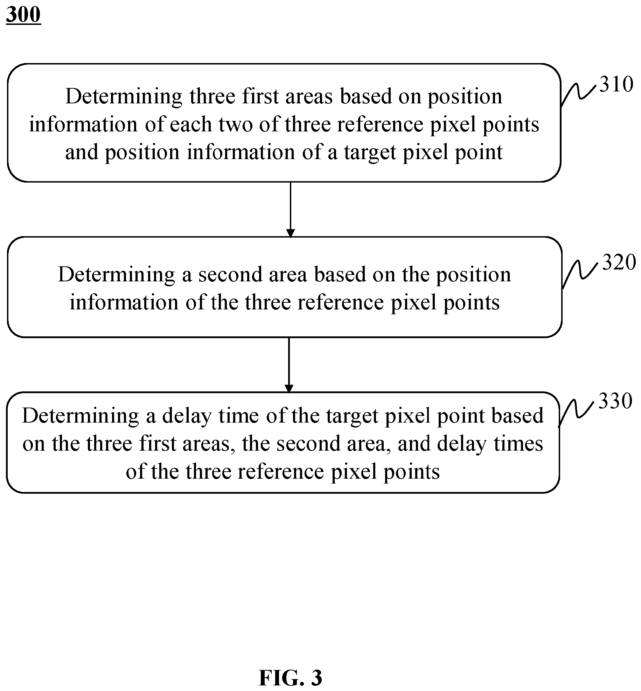

In some embodiments, when the target pixel point is located within a triangular region enclosed by three reference pixel points, the geometric information may include area information of a region formed by the target pixel point and the three reference pixel points. The processor may determine three first areas based on position information of any two of the three reference pixel points and the position information of the target pixel point; determine a second area based on the position information of the three reference pixel points; and determine the delay time of the target pixel point based on the three first areas, the second area, and the delay times of the three reference pixel points. For more details on this part, please refer to FIG. 3 and its related description.

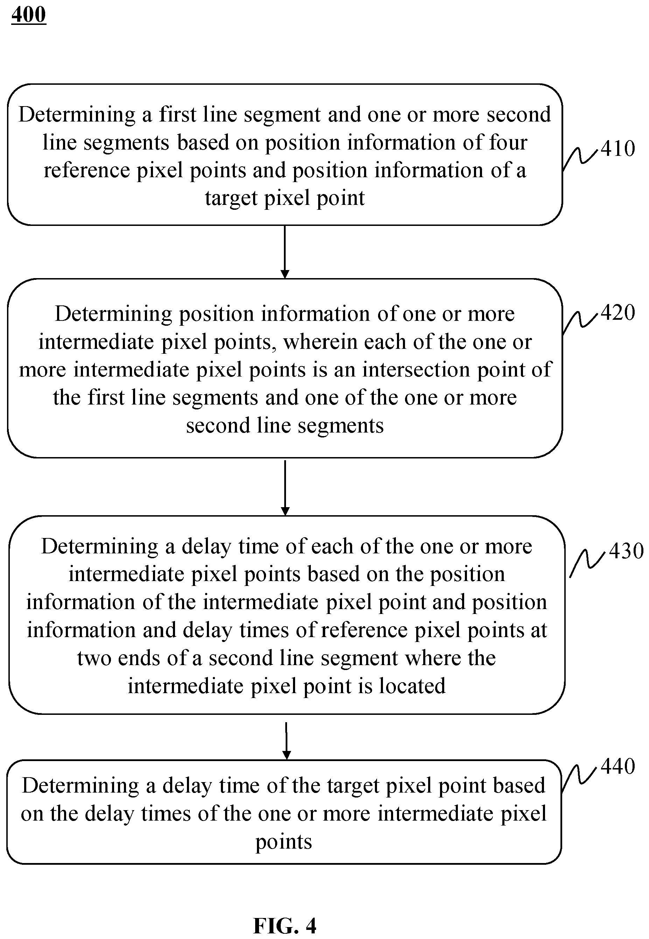

In some embodiments, when the target pixel point is located within a quadrilateral region enclosed by four reference pixel points, the geometric information may include line segment information of line segments between the target pixel point and the four reference pixel points. The processor may determine a first line segment and one or more second line segments based on position information of the four reference pixel points and the position information of the target pixel point. The first line segment passes through the target pixel point and intersects with the one or more second line segments, and each of the one or more second line segments connects a reference pixel point in the first effective region with a reference pixel point in the second effective region. The processor may then determine position information of one or more intermediate pixel points. Each of the one or more intermediate pixel points is an intersection point of the first line segment and one of the one or more second line segments. For each of the one or more intermediate pixel points, the processor may determine a delay time of the intermediate pixel point based on the position information of the intermediate pixel point and the position information and delay times of reference pixel points at two ends of the second line segment where the intermediate pixel point is located. Finally, the processor may determine the delay time of the target pixel point based on the delay times of the one or more intermediate pixel points. For more details on this part, please refer to FIG. 4 and its related description.

It should be noted that the processor may need to determine or predefine a shape of the region formed by the reference pixel points to determine the geometric information between the target pixel point and the reference pixel points, and further determine the delay time of the target pixel point. The delay time of the target pixel point is not directly related to a count of the reference pixel points. For example, when an emission corresponds to four reference pixel points, the target pixel point may be located within a triangular region enclosed by three of the four reference pixel points or within a quadrilateral region enclosed by all of the four reference pixel points. The processor may, as needed or predefined, choose to determine the area information between the target pixel point and the three reference pixel points or the line segment information between the target pixel point and the four reference pixel points, and further determine the delay time of the target pixel point. As another example, when an emission corresponds to three reference pixel points, the processor may determine the area information between the target pixel point and the three reference pixel points and further determine the delay time of the target pixel point. More descriptions of the determination of the delay time of the target pixel point based on the area information between the target pixel point and the three reference pixel points may be found in FIG. 3 and the related descriptions thereof. Alternatively, the processor may determine the line segment information between the target pixel point and the three reference pixel points and further determine the delay time of the target pixel point. In other words, the processor may determine a line segment formed by the target pixel point and one of the three reference pixel points, extend the line segment to obtain an intersection point between the extension of the line segment with a line segment formed by the other two reference pixel points among the three reference pixel points, determine the delay time of the intersection point based on the delay times of the two reference pixel points, and further determine the delay time of the target pixel point based on the delay time of the intersection point and the delay time of the other two reference pixel points. More descriptions of the determination of the delay time of the target pixel point based on line segment information may be found in FIGs. 4-6 and the related descriptions thereof.

In some embodiments of the present disclosure, by indirectly determining the delay time of each of the one or more target pixel points in the artifact region, which has a relatively low imaging quality, using the reference pixel points from the effective region with a relatively high imaging quality, the accuracy of the delay times of the artifact pixel points in the artifact region is improved.

In 240, the processor generates an ultrasonic image based on the delay time of each pixel point in the effective region and the delay time of each of the one or more target pixel points in the artifact region.

In some embodiments, the processor may extract an echo signal corresponding to each receiving channel at a corresponding time based on the delay time of each pixel point in the effective region and the delay time of the one or more target pixel points in the artifact region corresponding to each emission during ultrasonic imaging. The processor may then superimpose the echo signals corresponding to receiving channels at corresponding times to achieve dynamic focusing, thereby reconstructing and generating the ultrasonic image.

In some embodiments of the present disclosure, by indirectly calculating the delay times of the pixel points in the artifact region using the delay time of each pixel point in the effective region, and then generating the ultrasonic image based on the delay time of each pixel point in the effective region and the delay time of each pixel point in the artifact region, the difficulty in delay time calculations near the focal point in focused wave imaging based on the point source model is effectively and simply resolved, thereby achieving smooth transitions at hard boundaries, effectively eliminating imaging artifacts, and improving imaging quality.

In some embodiments, when the target pixel point is located within a triangular region enclosed by three reference pixel points, the processor may determine the delay time of the target pixel point based on the delay times of the three reference pixel points, the position information of the three reference pixel points, and the position information of the target pixel point.

In some embodiments, if at least one of the one or more target pixel points is located within a triangular region enclosed by three reference pixel points, then each of the first effective region and the second effective region in the effective region includes at least one of the three reference pixel points. For example, if the delay times of reference pixel points 1 to 3 are used to determine the delay time of target pixel point 1, each of the first effective region and the second effective region includes at least one of reference pixel points 1 to 3 (e.g., as shown in FIG. 7B, if reference pixel points (1), (2), and (5) are used to determine target pixel point (6), then the first effective region includes reference pixel points (1) and (5), and the second effective region includes reference pixel point (2), or the first effective region includes reference pixel point (1), and the second effective region includes reference pixel points (2) and (5)).

In some embodiments, the processor may perform modeling or use various data analysis algorithms, such as regression analysis, discriminant analysis, etc., to analyze and process the delay times of the three reference pixel points, the position information of the three reference pixel points, and the position information of the target pixel point, thereby determining the delay time of the target pixel point.

FIG. 3 is a flowchart of an exemplary process for determining a delay time of each of one or more target pixel points according to some embodiments of the present disclosure. As shown in FIG. 3, process 300 may include the following operations. In some embodiments, one or more operations of process 300 illustrated in FIG. 3 may be implemented in an ultrasonic imaging device. For example, process 300 shown in FIG. 3 may be stored in the form of instructions in a storage device and invoked and/or executed by a processor.

In 310, the processor determines three first areas based on position information of each two of three reference pixel points and position information of the target pixel point.

The first area refers to an area of a region enclosed by two reference pixel points and the target pixel point. As shown in FIG. 9A, A, B, and C denote three reference pixel points, and P is the target pixel point located within the triangular region formed by points A, B, and C. Areas of triangles $\triangle ABP$, $\triangle ACP$, and $\triangle BCP$ are the three first areas, denoted as $S_{\triangle ABP}$, $S_{\triangle ACP}$, and $S_{\triangle BCP}$, respectively.

In some embodiments, when the target pixel point is located within the triangular region formed by the three reference pixel points, the processor may calculate the first area $S_{\triangle ABP}$ using Equation (1), based on the position information of two of the three reference pixel points and the position information of the target pixel point. Equation (1) is as follows:

$$S_{\Delta ABP} = 0.5 * |x_1 * (y_2 - y) + x_2 * (y - y_1) + x * (y_1 - y_2)| \qquad (1),$$

wherein coordinates of points A, B, C, and P are $(x_1, y_1)$, $(x_2, y_2)$, $(x_3, y_3)$, and $(x, y)$, respectively. $S_{\Delta ACP}$ and $S_{\Delta BCP}$ may be calculated similarly, and thus will not be elaborated further.

In 320, the processor determines a second area based on the position information of the three reference pixel points.

The second area refers to an area of a region enclosed by the multiple reference pixel points. As shown in FIG. 9A, the second area may be an area, denoted as $S_{\Delta ABC}$, of a triangle $\Delta ABC$ formed by reference pixel points A, B, and C.

In some embodiments, the processor may calculate the second area $S_{\Delta ABC}$ based on the position information of the three reference pixel points using Equation (2) as followings:

$$S_{\Delta ABC} = 0.5 * |x_1 * (y_2 - y_3) + x_2 * (y_3 - y_1) + x_3 * (y_1 - y_2)| \qquad (2).$$

wherein the coordinates of points A, B, and C are $(x_1, y_1)$, $(x_2, y_2)$, and $(x_3, y_3)$, respectively.

In 330, the processor determines the delay time of the target pixel point based on the three first areas, the second area, and the delay times of the three reference pixel points.

In some embodiments, the processor may determine the delay time of the target pixel point by modeling or using various data analysis algorithms, such as regression analysis, discriminant analysis, etc., based on the three first areas, the second area, and the delay times of the three reference pixel points. For example, the processor may input the three first areas, the second area, and the delay times of the three reference pixel points into a trained computational model, and determine the delay time of the target pixel point based on the computational model.

In some embodiments, for each first area of the three first area, the processor may determine an area ratio of the first area to the second area, and determine a product of the area ratio corresponding to the first area and the delay time of a reference pixel point corresponding to the first area. Then the processor may determine the delay time of the target pixel point based on products corresponding to the three first areas.

The area ratio refers to a ratio of the first area to the second area.

The reference pixel point corresponding to the first area refers to a reference pixel point that is not part of the first area. For example, the reference pixel point corresponding to the first area $S_{\Delta ABP}$ is reference pixel point C, which is not part of the first area.

In some embodiments, for each of the three first areas, the processor may multiply the area ratio corresponding to the first area by the delay time of the reference pixel point corresponding to the first area, and determine a product result.

In some embodiments, the processor may determine a sum of the products corresponding to the three first areas as the delay time of the target pixel point using Equation (3):

$$t_p = \frac{S_{\Delta ABP}}{S_{\Delta ABC}} \cdot t_C + \frac{S_{\Delta ACP}}{S_{\Delta ABC}} \cdot t_B + \frac{S_{\Delta BCP}}{S_{\Delta ABC}} \cdot t_A \qquad (3),$$

wherein $\frac{S_{\Delta ABP}}{S_{\Delta ABC}}$, $\frac{S_{\Delta ACP}}{S_{\Delta ABC}}$, and $\frac{S_{\Delta BCP}}{S_{\Delta ABC}}$ denote the area ratio of the first area $S_{\Delta ABP}$ to the second area $S_{\Delta ABC}$, the area ratio of the first area $S_{\Delta ACP}$ to the second area $S_{\Delta ABC}$, and the area ratio of the first area $S_{\Delta BCP}$ to the second area $S_{\Delta ABC}$, respectively. $t_C$, $t_B$, and $t_A$ represent the delay times of the reference pixel points C, B, and A corresponding to

$\frac{S_{\Delta ABP}}{S_{\Delta ABC}}$, $\frac{S_{\Delta ACP}}{S_{\Delta ABC}}$, and $\frac{S_{\Delta BCP}}{S_{\Delta ABC}}$, respectively. $t_p$ denotes the delay time of the target pixel point P.

In some embodiments, the processor may determine weights of the reference pixel points using a proportional relationship between each of the three first areas and the second area, and then optimize and adjust the weights. For example, the processor may calculate an average weight of reference pixel points A, B, and C, or round up the weights of the reference pixel points A, B, and C, and then determine the delay time of the target pixel point P by performing a weighted summation of the delay times of the reference pixel points.

Some embodiments of the present disclosure use the proportional relationship between each of the three first areas and the second area to determine the weights of the reference pixel points forming the first areas, and further perform a weighted summation of the delay times of the reference pixel points based on the weights. This approach reasonably adjusts the importance of the delay time of each reference pixel point, making the indirectly determined delay time of the target pixel point in the artifact region more accurate. Additionally, this approach expands the area of the artifact region, thereby avoiding artifact issues.

In some embodiments of the present disclosure, the delay time of the target pixel point in the artifact region is indirectly determined based on the first areas, the second area, and the delay times of the reference pixel points. This approach improves the accuracy of the delay time of the target pixel point and expands the imaging region near the focal point, thereby preventing dark zone artifacts in the artifact region caused by the imaging region from narrowing near the focal point.

In some embodiments of the present disclosure, three reference pixel points are selected, allowing for the simplest and most effective improvement in the accuracy of the delay time of the target pixel point within the triangular region formed by the three reference pixel points, using a minimum count of reference pixel points and their corresponding delay times.

In some embodiments, when the target pixel point is located within a quadrilateral region enclosed by four reference pixel points, the processor may determine the delay time of the target pixel point based on delay times of the four reference pixel points, position information of the four reference pixel points, and the position information of the target pixel point.

In some embodiments, if at least one of the one or more target pixel points is located within a quadrilateral region enclosed by four reference pixel points, then each of the first effective region and the second effective region includes at least one of the four reference pixel points. For example, if reference pixel points 1 to 4 are used to determine target pixel point 1, then each of the first effective region and the second effective region includes at least one of reference pixel points 1 to 4 (e.g., the first effective region includes reference pixel points 1 and 2, and the second effective region includes reference pixel points 3 and 4, or the first effective region includes reference pixel points 1 to 3, and the second effective region includes reference pixel point 4). For example, as shown in FIG. 7C, if reference pixel points (7), (8), (9), and (10) are used to determine target pixel point (11), then the first effective region may include reference pixel points (7) and (8), and the second effective region may include reference pixel points (9) and (10).

In some embodiments, the processor may perform modeling or use various data analysis algorithms, such as regression analysis, discriminant analysis, etc., to analyze and process the delay times of the four reference pixel points, the position information of the four reference pixel points, and the position information of the target pixel point, thereby determining the delay time of the target pixel point.

FIG. 4 is a flowchart of another exemplary process for determining a delay time of each of one or more target pixel points according to some embodiments of the present disclosure. As shown in FIG. 4, process 400 may include the following operations. In some embodiments, one or more operations of process 400 illustrated in FIG. 4 may be implemented in an ultrasonic imaging device. For example, process 400 shown in FIG. 4 may be stored in the form of instructions in a storage device and invoked and/or executed by a processor.

In 410, the processor determines a first line segment and one or more second line segments based on position information of four reference pixel points and position information of the target pixel point.

The first line segment refers to a line segment that passes through the target pixel point and intersects with the one or more second line segments. As shown in FIG. 9B, a straight line is drawn through point P, intersecting with second line segments AB and CD at points E and F, respectively. Thus, a line segment EF is a first line segment, and points E and F are intermediate pixel points. In some embodiments, the processor may select one line segment from multiple line segments that pass through the target pixel point and intersect with the one or more second line segments as the first line segment according to predefined rules. For example, the processor may select a line segment parallel to the array element from the aforementioned multiple line segments as the first line segment.

A second line segment refers to a line segment formed by connecting a reference pixel point in the first effective region of the effective region with a reference pixel point in the second effective region of the effective region. As shown in FIG. 9B, A, B, C, and D are four reference pixel points, and point P is the target pixel point in the artifact region, located within the quadrilateral region enclosed by the reference pixel points A, B, C, and D. The processor connects points A and B to form a line segment AB and connects points C and D to form a line segment CD. Thus, the line segments AB and CD are second line segments.

More descriptions of the first effective region and the second effective region may be found in FIG. 2 and the related descriptions thereof.

In 420, the processor determines position information of one or more intermediate pixel points. Each of the one or more intermediate pixel points is an intersection point of the first line segment and one of the one or more second line segments.

The one or more intermediate pixel points may be used to assist in determining the target pixel point. In some embodiments, a count of the one or more intermediate pixel points is two.

The position information of the one or more intermediate pixel points refers to data information reflecting location(s) of the one or more intermediate pixel points.

In some embodiments, the processor may determine the position information of the one or more intermediate pixel points in a coordinate system of the imaging device based on the position information of the four reference pixel points and the position information of the target pixel point, as well as a positional relationship between the one or more intermediate pixel points, the four reference pixel points, and the target pixel point. More descriptions of the position information of the reference pixel points and the position information of the target pixel point may be found in FIG. 2 and the related descriptions thereof.

In 430, the processor determines a delay time of each of the one or more intermediate pixel points based on the position information of the intermediate pixel point and position information and delay times of reference pixel points at two ends of the second line segment where the intermediate pixel point is located.

In some embodiments, for each of the one or more intermediate pixel points, the processor may determine the delay time of the intermediate pixel point in various ways based on the position information of the intermediate pixel point and delay times of reference pixel points at two ends of the second line segment where the intermediate pixel point is located. For example, the processor may determine the delay time of the intermediate pixel point through interpolation based on the delay times of the reference pixel points at the two ends of the second line segment where the intermediate pixel point is located.

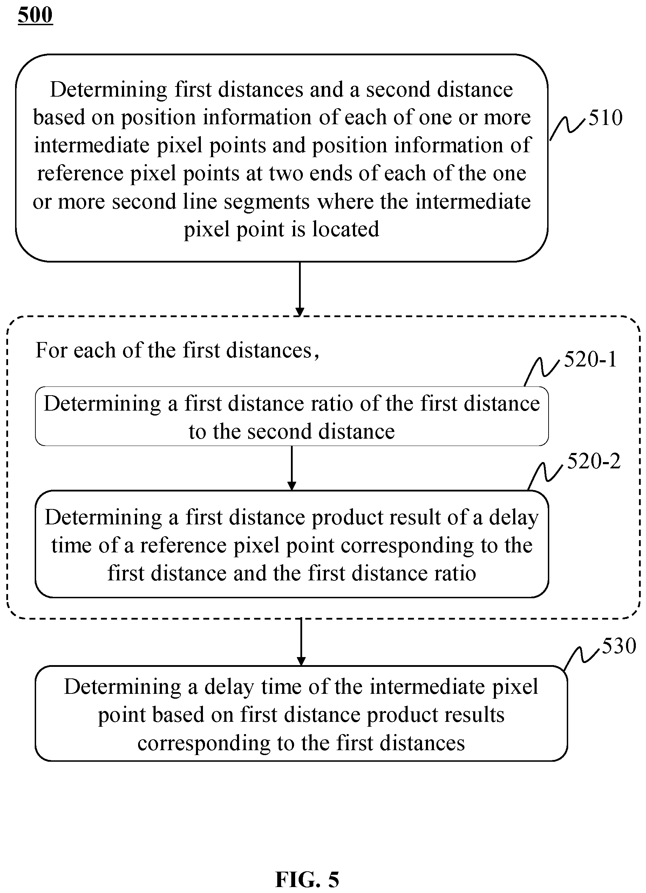

In some embodiments, the processor may determine first distances and a second distance based on the position information of the intermediate pixel point and the position information of the reference pixel points at the two ends of each of the one or more second line segments where the intermediate pixel point is located. The processor may determine, for each of the first distances, a first distance ratio of the first distance to the second distance; determine a first distance product result of the delay time of the reference pixel point corresponding to the first distance and the first distance ratio; and determine the delay time of the intermediate pixel point based on first distance product results corresponding to the first distances. More descriptions of the above embodiment may be found in FIG. 5 and the related descriptions thereof.

In 440, the processor determines the delay time of the target pixel point based on the delay times of the one or more intermediate pixel points.

In some embodiments, the processor may determine the delay time of the target pixel point in various ways based on the delay times of the one or more intermediate pixel points. For example, the processor may input the delay times of the one or more intermediate pixel points into a trained machine learning model to output the delay time of the target pixel point.

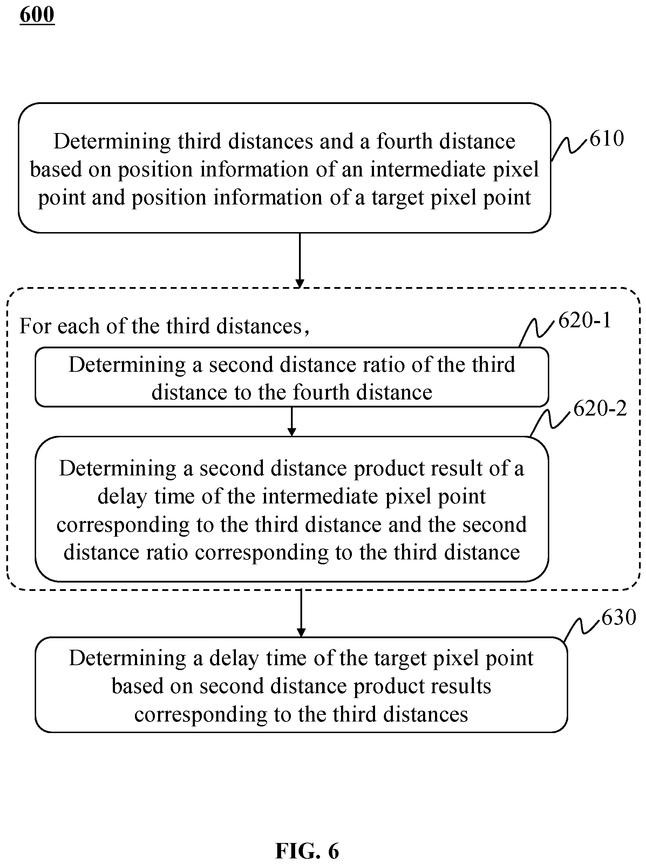

In some embodiments, the processor may determine third distances and a fourth distance based on the position information of the intermediate pixel point and the position information of the target pixel point. The processor may determine, for each of the third distances, a second distance ratio of the third distance to the fourth distance; determine a second distance product result of the delay time of the intermediate pixel point corresponding to the third distance and the second distance ratio corresponding to the third distance; and determine the delay time of the target pixel point based on second distance product results corresponding to the third distances. More descriptions of the above embodiment may be found in FIG. 6 and the related descriptions thereof.

In some embodiments of the present disclosure, by introducing one or more intermediate pixel points and indirectly determining the delay time of the target pixel point based on the delay times of the one or more intermediate pixel points, the accuracy of the delay time of the target pixel point in the artifact region is improved. Additionally, the area of the artifact region is expanded, thereby preventing the imaging region from narrowing near the focal point, effectively eliminating imaging artifacts, and improving imaging quality.

In some embodiments of the present disclosure, by using four reference pixel points and the delay times corresponding to the four reference pixel points, the accuracy of the delay time of the target pixel point within the quadrilateral region formed by the four reference pixel points is improved, thereby ensuring smooth transitions in calculated emission delays.

FIG. 5 is a flowchart of an exemplary process for determining a delay time of each of one or more intermediate pixel points according to some embodiments of the present disclosure. As shown in FIG. 5, process 500 may include the following operations. In some embodiments, one or more operations of process 500 illustrated in FIG. 5 may be implemented in an ultrasonic imaging device. For example, process 500 shown in FIG. 5 may be stored in the form of instructions in a storage device and invoked and/or executed by a processor.

In 510, the processor determines first distances and a second distance based on positional information of an intermediate pixel point and positional information of reference pixel points at two ends of a second line segment where the intermediate pixel point is located.

A first distance corresponding to an intermediate pixel point refers to a distance between the intermediate pixel point and a reference pixel point at an end of the second line segment where the intermediate pixel point is located. For example, the first distances may include Euclidean distances between the intermediate pixel point on the second line segment and the reference pixel points at the two ends of the second line segment where the intermediate pixel point is located. Each of the one or more intermediate pixel points may correspond to two first distances. As shown in FIG. 9B, the first distances corresponding to an intermediate pixel point E include a Euclidean distance between point E and point A and a Euclidean distance between point E and point B. The first distances corresponding to an intermediate pixel point F include a Euclidean distance between point F and point C and a Euclidean distance between point F and point D. The first distances $D_{AE}$, $D_{BE}$, $D_{CF}$, and $D_{DF}$ may be the Euclidean distances of line segments AE, BE, CF, and DF, respectively.

In some embodiments, for each of the one or more intermediate pixel points, the processor may determine the first distances based on positional coordinates of the intermediate pixel point and reference pixel points at two ends of the second line segment where the intermediate pixel point is located, using a distance equation. Taking $D_{AE}$ as an example, $D_{AE}$ is determined using Equation (4), and $D_{BE}$, $D_{CF}$, and $D_{DF}$ may be determined in the same manner, which will not be repeated here.

$$D_{AE} = \sqrt{(x_5 - x_1)^2 + (y_5 - y_1)^2} \qquad (4),$$

wherein $(x_1, y_1)$ and $(x_5, y_5)$ represent the coordinates of points A and E, respectively.

The second distance corresponding to an intermediate pixel point refers to a distance between the reference pixel points at the two ends of the second line segment where the intermediate pixel point is located. For example, the second distance may be a Euclidean distance between the reference pixel points at the two ends of the second line segment where the intermediate pixel point is located. As shown in FIG. 9B, the second distance corresponding to an intermediate pixel point E is a Euclidean distance between points A and B, and the second distance corresponding to an intermediate pixel point F is a Euclidean distance between points C and D. The second distances $D_{AB}$ and $D_{CD}$ may be the Euclidean distances of line segments AB and CD, respectively.

In some embodiments, the processor may determine the second distance based on the positional coordinates of the reference pixel points at the two ends of the second line segment using a distance equation similar to Equation (4) and will not be repeated here.

For each of the first distances, the processor may perform the following operations 520-1 and 520-2.

In 520-1, the processor determines a first distance ratio of the first distance to the second distance.

The first distance ratio corresponding to a first distance refers to a ratio of the first distance to the second distance corresponding to the second line segment where the intermediate pixel point is located. As shown in FIG. 9B, the first distance ratios may include $\dfrac{D_{BE}}{D_{AB}}$, $\dfrac{D_{AE}}{D_{AB}}$, $\dfrac{D_{DF}}{D_{CD}}$, and $\dfrac{D_{CF}}{D_{CD}}$.

The processor may determine the first distance ratio corresponding to a first distance by calculating a ratio of the first distance to the second distance corresponding to the second line segment where the intermediate pixel point is located.

In 520-2, the processor determines a first distance product result of the delay time of a reference pixel point corresponding to the first distance and the first distance ratio.

The reference pixel corresponding to a first distance refers to the other reference pixel on the second line segment corresponding to the first distance that is not involved in forming the first distance. For example, the reference pixel corresponding to the first distance $D_{BE}$ is a reference pixel point A, which is located on the second line segment AB corresponding to the first distance and is not involved in forming the first distance $D_{BE}$.

The first distance product result corresponding to a first distance refers to a product of the delay time of the reference pixel corresponding to the first distance and the first distance ratio corresponding to the first distance. As shown in FIG. 9B, the first distance product results may include $\dfrac{D_{BE}}{D_{AB}} * t_A$, $\dfrac{D_{AE}}{D_{AB}} * t_B$, $\dfrac{D_{DF}}{D_{CD}} * t_C$, and $\dfrac{D_{CF}}{D_{CD}} * t_D$, wherein $t_A$ denotes the delay time of the reference pixel point A corresponding to $D_{BE}$, $t_B$ denotes the delay time of a reference pixel point B corresponding to $D_{AE}$, $t_C$ denotes the delay time of a reference pixel point C corresponding to $D_{DF}$, and $t_D$ denotes the delay time of a reference pixel point D corresponding to $D_{CF}$.

In 530, the processor determines the delay time of the intermediate pixel point based on the first distance product results corresponding to the first distances.

In some embodiments, the processor may determine the delay time of the intermediate pixel point using summation Equations (5) and (6) based on the first distance product results.

$$t_E = \frac{D_{BE}}{D_{AB}} * t_A + \frac{D_{AE}}{D_{AB}} * t_B \qquad (5),$$

$$t_F = \frac{D_{DF}}{D_{CD}} * t_C + \frac{D_{CF}}{D_{CD}} * t_D \qquad (6),$$

wherein $t_E$ and $t_F$ denote the delay times of intermediate pixel points E and F, respectively.

In some embodiments, the processor may determine the delay time of the intermediate pixel point using other manners. For example, the processor may determine a weight of the intermediate pixel point E based on a proportional relationship between line segments AE and BE, optimize and adjust the weight of the intermediate pixel point E (e.g., by calculating an average weight of the intermediate pixel point E or rounding up the weight of the intermediate pixel point E), and then determine the delay time $t_E$ of the intermediate pixel point E through a weighted summation based on the delay times of the reference pixel points A and B. The delay time $t_F$ of the intermediate pixel point F may be determined in a similar manner.

In some embodiments of the present disclosure, by summing the product results of the first distance ratios and the delay time of the corresponding reference pixel point, the delay time of the intermediate pixel point is determined, allowing for convenient and efficient interpolation of the delay time of the target pixel point even in cases involving four reference pixel points, thereby improving the accuracy of the delay time of the target pixel point.

FIG. 6 is a flowchart of an exemplary process for determining a delay time of each of one or more target pixel points according to some embodiments of the present disclosure. As shown in FIG. 6, process 600 may include the following operations. In some embodiments, one or more operations of process 600 illustrated in FIG. 6 may be implemented in an ultrasonic imaging device. For example, process 600 shown in FIG. 6 may be stored in the form of instructions in a storage device and invoked and/or executed by a processor.

In 610, the processor determines third distances and a fourth distance based on positional information of one or more intermediate pixel points and positional information of the target pixel point.

A third distance corresponding to a target pixel point refers to a distance between the target pixel point and an intermediate pixel point at an end of a first line segment. For example, the third distances may include Euclidean distances between the target pixel point and the intermediate pixel points at two ends of the first line segment. Each of one or more target pixel points may correspond to two third distances. As shown in FIG. 9B, the third distances corresponding to a target pixel point P are a Euclidean distance between point E and point P and a Euclidean distance between point F and point P. The third distances $D_{EP}$ and $D_{FP}$ may be Euclidean distances of line segments EP and FP, respectively.

The fourth distance refers to a distance between intermediate pixel points at two ends of the first line segment. For example, the fourth distance may be a Euclidean distance between the intermediate pixel points at the two ends of the first line segment. As shown in FIG. 9B, the fourth distance corresponding to the target pixel point P is a Euclidean distance between points E and F, and the fourth distance $D_{EF}$ may be a Euclidean distance of the line segment EF.

In some embodiments, the processor may determine the third distances based on positional coordinates of the target pixel point and the intermediate pixel points at the two ends of the first line segment using a distance equation, and determine the fourth distance based on positional coordinates of the intermediate pixel points at the two ends of the first line segment using a distance equation. The third distances $D_{EP}$ and $D_{FP}$ and the fourth distance $D_{EF}$ may be calculated in a manner similar to the calculation of the first distance $D_{AE}$ using Equation (4), and will not be repeated here.

For each of the third distances, the processor may perform the following operations 620-1 and 620-2.

In 620-1, the processor determines a second distance ratio of the third distance to the fourth distance.

The second distance ratio corresponding to a third distance refers to a ratio of the third distance to the fourth distance. As shown in FIG. 9B, the second distance ratios may include $\dfrac{D_{EP}}{D_{EF}}$ and $\dfrac{D_{FP}}{D_{EF}}$.

In some embodiments, the processor may directly determine the second distance ratio through calculation.

In 620-2, the processor determines a second distance product result of the delay time of the intermediate pixel point corresponding to the third distance and the second distance ratio corresponding to the third distance.

The intermediate pixel point corresponding to a third distance refers to the other intermediate pixel point on the first line segment corresponding to the third distance that is not involved in forming the third distance. For example, the intermediate pixel point corresponding to the third distance $D_{EP}$ is the intermediate pixel point F, which is located on the first line segment EF corresponding to the third distance and is not involved in forming the third distance $D_{EP}$.

The second distance product result corresponding to a third distance refers to the product of the delay time of the intermediate pixel point corresponding to the third distance and the second distance ratio corresponding to the third distance. As shown in FIG. 9B, the two second distance product results corresponding to the two third distances $D_{EP}$ and $D_{FP}$ include $\dfrac{D_{EP}}{D_{EF}} * t_F$ and $\dfrac{D_{FP}}{D_{EF}} * t_E$, wherein $t_F$ denotes the delay time of the intermediate pixel point F corresponding to the third distance $D_{EP}$, and $t_E$ denotes the delay time of the intermediate pixel point E corresponding to the third distance $D_{FP}$.

In 630, the processor determines the delay time of the target pixel point based on the second distance product results corresponding to the third distances.

In some embodiments, the processor may determine the delay time of the intermediate pixel point using a summation equation based on the second distance product results. The summation equation may include Equation (7):

$$t_P = \frac{D_{EP}}{D_{EF}} * t_F + \frac{D_{FP}}{D_{EF}} * t_E \qquad (7),$$

Wherein $t_p$ denotes the delay time of the target pixel point P.

In some embodiments, combining Equations (5), (6), and (7), the processor may determine the delay time of the target pixel point using Equation (8):

$$t_P = \frac{D_{EP}}{D_{EF}} * (\frac{D_{DF}}{D_{CD}} * t_C + \frac{D_{CF}}{D_{CD}} * t_D) + \frac{D_{FP}}{D_{EF}} * (\frac{D_{BE}}{D_{AB}} * t_A + \frac{D_{AE}}{D_{AB}} * t_B) \qquad (8).$$

In some embodiments of the present disclosure, by summing the products of the delay times of the intermediate pixel points corresponding to the third distances and the second distance ratios corresponding to the third distances, the delay time of the target pixel point is determined based on the intermediate pixel points, thereby enabling the determination of the delay time of the target pixel point based on four reference pixel points, and avoiding artifacts caused by the narrowing of the imaging region near the focal point.

It should be noted that the descriptions of the processes above are provided for illustrative and explanatory purposes only and do not limit the scope of the present disclosure. For those skilled in the art, various modifications and changes to the processes may be made under the guidance of the present disclosure. However, these modifications and changes still fall within the scope of the present disclosure.

One or more embodiments of the present disclosure provide a device for ultrasonic imaging. The device includes at least one processor and at least one storage device. The at least one storage device is configured to store computer instructions. The at least one processor is configured to execute at least a portion of the computer instructions to implement the method for ultrasonic imaging described in any of the above embodiments of the present disclosure.

One or more embodiments of the present disclosure provide a non-transitory computer-readable storage medium storing computer instructions. When a computer reads the computer instructions from the storage medium, the computer executes the method for ultrasonic imaging described in any of the above embodiments of the present disclosure.

Having thus described the basic concepts, it may be rather apparent to those skilled in the art after reading this detailed disclosure that the foregoing detailed disclosure is intended to be presented as illustrative example and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of the present disclosure.

Moreover, certain terminology has been configured to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations, therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software-only solution, e.g., an installation on an existing server or mobile device.

Similarly, it should be noted that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments lie in less than all features of a single foregoing disclosed embodiment.

Some embodiments use numbers to describe the count of components, and attributes, and it should be understood that such numbers used in the description of the embodiments are modified in some examples by the modifiers "about," "approximately," or "generally." Unless otherwise stated, "about," "approximately," or "generally" indicates that a variation of $\pm$ 20% is permitted. Accordingly, in some embodiments, the numerical parameters used in the present disclosure and claims are approximations, which may change depending on the desired characteristics of the individual embodiment. In some embodiments, the numeric parameters should be considered with the specified significant figures and be rounded to a general number of decimal places. Although the numerical domains and parameters configured to confirm the breadth of their ranges in some embodiments of the present disclosure are approximations, in specific embodiments such values are set as precisely as possible within the feasible range.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrating of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for ultrasonic imaging implemented on a processor, the method comprising:

   determining a delay time of each pixel point in an effective region based on an echo signal of a focused wave;
   determining reference pixel points from the effective region and delay times corresponding to the reference pixel points;
   determining a delay time of each of one or more target pixel points in an artifact region based on the reference pixel points and the delay times corresponding to the reference pixel points, wherein the one or more target pixel points are located within a region enclosed by the reference pixel points; and
   generating an ultrasonic image based on the delay time of each pixel point in the effective region and the delay time of each of the one or more target pixel points in the artifact region.

2. The method of claim 1, wherein the effective region includes a propagation region of the focused wave, and the artifact region is a region other than the effective region within an imaging region corresponding to the focused wave.

3. The method of claim 1, wherein the determining a delay time of each pixel point in an effective region based on an echo signal of a focused wave includes:
   determining the delay time of the each pixel point in the effective region by processing the echo signal of the focused wave using a point source model.

4. The method of claim 1, wherein the one or more target pixel points in the artifact region is determined according to operations including:
   for each emission,

   determining one or more target overlap points between an effective region of the each emission and an effective region of an adjacent emission, wherein the one or more target overlap points are within an overlap region between the effective region of the each emission and the effective region of the adjacent emission and have a smallest distance to a focal pixel point of the each emission;
   determining a target region based on the focal pixel point and the one or more target overlap points; and
   determining the one or more target pixel points in the artifact region based on the target region, wherein the one or more target pixel points in the artifact region at least include all pixel points within the target region.

5. The method of claim 4, wherein the one or more target pixel points further include other pixel points in the artifact region outside the target region, and
   for each emission, there is a 30% to 80% overlap between one or more target pixel points corresponding to the each emission and one or more target pixel points corresponding to the adjacent emission.

6. The method of claim 1, wherein the determining a delay time of each of one or more target pixel points in an artifact region based on the reference pixel points and the delay times corresponding to the reference pixel points includes:
   for each of the one or more target pixel points,
   determining a delay time of the target pixel point based on delay times of the reference pixel points, position information of the reference pixel points, and position information of the target pixel point.

7. The method of claim 6, wherein the determining a delay time of the target pixel point based on delay times of the reference pixel points, position information of the reference pixel points, and position information of the target pixel point includes:

determining geometric information between the target pixel point and the reference pixel points based on the position information of the reference pixel points and the position information of the target pixel point; and determining the delay time of the target pixel point based on the geometric information and the position information of the reference pixel points.

8. The method of claim 7, wherein when the reference pixel points are located within a triangular region enclosed by three reference pixel points, the geometric information includes area information of a region formed by the target pixel point and the three reference pixel points,

the determining geometric information between the target pixel point and the reference pixel points based on the position information of the reference pixel points and the position information of the target pixel point includes:

determining three first areas based on position information of each two of the three reference pixel points and the position information of the target pixel point; and determining a second area based on the position information of the three reference pixel points;

the determining the delay time of the target pixel point based on the geometric information and the position information of the reference pixel points includes:
determining the delay time of the target pixel point based on the three first areas, the second area, and the delay times of the three reference pixel points.

9. The method of claim 8, wherein the determining the delay time of the target pixel point based on the three first areas, the second area, and the delay times of the three reference pixel points includes:

for each first area of the three first areas,

determining an area ratio of the each first area to the second area; determining a product of the area ratio corresponding to the each first area and the delay time of the reference pixel point corresponding to the each first area; and

determining the delay time of the target pixel point based on products corresponding to the three first areas.

10. The method of claim 7, wherein the effective region is composed of a first effective region and a second effective region formed by a propagation of the focused wave, and when the target pixel point is located within a quadrilateral region enclosed by four reference pixel points, the geometric information includes line segment information of line segments between the target pixel point and the four reference pixel points,

the determining geometric information between the target pixel point and the reference pixel points based on the position information of the reference pixel points and the position information of the target pixel point includes:

determining a first line segment and one or more second line segments based on position information of the four reference pixel points and the position information of the target pixel point, wherein the first line segment passes through the target pixel point and intersects with the one or more second line segments, and each of the one or more second line segments connects a reference pixel point in the first effective region with a reference pixel point in the second effective region;

the determining the delay time of the target pixel point based on the geometric information and the position information of the reference pixel points includes:

determining position information of one or more intermediate pixel points, wherein each of the one or more intermediate pixel points is an intersection point of the first line segment and the one or more second line segments;
determining a delay time of each of the one or more intermediate pixel points based on the position information of the intermediate pixel point and position information and delay times of reference pixel points at two ends of the second line segment where the intermediate pixel point is located; and

determining the delay time of the target pixel point based on the delay times of the one or more intermediate pixel points.

11. The method of claim 10, wherein the determining a delay time of each of the one or more intermediate pixel points based on the position information of the intermediate pixel point and position information and delay times of reference pixel points at two ends of the second line segment where the intermediate pixel point is located includes:

determining first distances and a second distance based on the position information of the intermediate pixel point and the position information of the reference pixel points at the two ends of each of the one or more second line segments where the intermediate pixel point is located, wherein each of the first distances is a distance between the intermediate pixel point and a reference pixel point at an end of the second line segment where the intermediate pixel point is located, and the second distance is a distance between the reference pixel points at the two ends of the second line segment;

for each of the first distances,

determining a first distance ratio of the first distance to the second distance;
determining a first distance product result of the delay time of the reference pixel point corresponding to the first distance and the first distance ratio; and

determining the delay time of the intermediate pixel point based on first distance product results corresponding to the first distances.

12. The method of claim 10, wherein the determining the delay time of the target pixel point based on the delay times of the one or more intermediate pixel points includes:

determining third distances and a fourth distance based on the position information of the intermediate pixel point and the position information of the target pixel point, wherein each of the third distances is a distance between the target pixel point and an intermediate pixel point at an end of the first line segment, and the fourth distance is a distance between intermediate pixel points at two ends of the first line segment;

for each of the third distances,

determining a second distance ratio of the third distance to the fourth distance;
determining a second distance product result of the delay time of the intermediate pixel point corresponding to the third distance and the second distance ratio corresponding to the third distance; and

determining the delay time of the target pixel point based on second distance product results corresponding to the third distances.

13. A system for ultrasonic imaging, the system comprising:

a first determination module, configured to determine a delay time of each pixel point in an effective region based on an echo signal of a focused wave;
a second determination module, configured to determine reference pixel points from the effective region and delay times corresponding to the reference pixel points;
a third determination module, configured to determine a delay time of each of one or more target pixel points in an artifact region based on the reference pixel points and the delay times corresponding to the reference pixel points, wherein the one or more target pixel points are located within a region enclosed by the reference pixel points; and
an image generation module, configured to generate an ultrasonic image based on the delay time of each pixel point in the effective region and the delay time of each of the one or more target pixel points in the artifact region.

14. The system of claim 13, wherein the first determination module is further configured to:
determine the delay time of the each pixel point in the effective region by processing the echo signal of the focused wave using a point source model.

15. The system of claim 13, wherein the third determination module is further configured to:

for each of the one or more target pixel points,

determine a delay time of the target pixel point based on delay times of the reference pixel points, position information of the reference pixel points, and position information of the target pixel point.

16. The system of claim 15, wherein the third determination module is further configured to:

determine geometric information between the target pixel point and the reference pixel points based on the position information of the reference pixel points and the position information of the target pixel point; and determine the delay time of the target pixel point based on the geometric information and the position information of the reference pixel points.

17. The system of claim 16, wherein when the reference pixel points are located within a triangular region enclosed by three reference pixel points, the geometric information includes area information of a region formed by the target pixel point and the three reference pixel points,
the third determination module is further configured to:

determine three first areas based on position information of any two of the three reference pixel points and the position information of the target pixel point;
determine a second area based on the position information of the three reference pixel points; and
determine the delay time of the target pixel point based on the three first areas, the second area, and the delay times of the three reference pixel points.

18. The system of claim 17, wherein the third determination module is further configured to:

for each first area of the three first areas,

determine an area ratio of the first area to the second area;
determine a product of the area ratio corresponding to the first area and the delay time of the reference pixel point corresponding to the first area; and

determine the delay time of the target pixel point based on products corresponding to the three first areas.

19. The system of claim 16, wherein the effective region is composed of a first effective region and a second effective region formed by a propagation of the focused wave, and when the target pixel point is located within a quadrilateral region enclosed by four reference pixel points, the geometric information includes line segment information of a region formed by the target pixel point and the four reference pixel points,
the third determination module is further configured to:

determine a first line segment and one or more second line segments based on position information of the four reference pixel points and the position information of the target pixel point, wherein the first line segment is a line segment that passes through the target pixel point and intersects with one of the one or more second line segments, and each of the one or more second line segments connects a reference pixel point in the first effective region with a reference pixel point in the second effective region;
determine position information of one or more intermediate pixel points, wherein each of the one or more intermediate pixel points is an intersection point of the first line segment and one of the one or more second line segments;
determine a delay time of each of the one or more intermediate pixel points based on the position information of the intermediate pixel point and position information and delay times of reference pixel points at two ends of the second line segment where the intermediate pixel point is located; and
determine the delay time of the target pixel point based on the delay times of the one or more intermediate pixel points.

20. The system of claim 19, wherein the third determination module is further configured to:

determine first distances and a second distance based on the position information of the intermediate pixel point and the position information of the reference pixel points at the two ends of each of the one or more second line segments where the intermediate pixel point is located, wherein each of the first distances is a distance between the intermediate pixel point and a reference pixel point at an end of the second line segment where the intermediate pixel point is located, and the second distance is a distance between the reference pixel points at the two ends of the second line segment;
for each of the first distances,

determine a first distance ratio of the first distance to the second distance;
determine a first distance product result of the delay time of the reference pixel point corresponding to the first distance and the first distance ratio; and

determine the delay time of the intermediate pixel point based on first distance product results corresponding to the first distances.

21. The system of claim 19, wherein the third determination module is further configured to:

determine third distances and a fourth distance based on the position information of the intermediate pixel point and the position information of the target pixel point, wherein each of the third distances is a distance between the target pixel point and an intermediate pixel point at an end of the first line segment, and the fourth distance is a distance between intermediate pixel points at two ends of the first line segment;
for each of the third distances,

determine a second distance ratio of the third distance to the fourth distance;
determine a second distance product result of the delay time of the intermediate pixel point corresponding to the third distance and the second distance ratio corresponding to the third distance; and

determine the delay time of the target pixel point based on second distance product results corresponding to the third distances.

22. An ultrasonic imaging device, comprising at least one processor and at least one storage device; wherein

the at least one storage device is configured to store computer instructions; and
the at least one processor is configured to execute at least a portion of the computer instructions to implement the method for ultrasonic imaging of any one of claims 1 to 12.

23. A non-transitory computer-readable storage medium storing computer instructions, wherein when a computer reads the computer instructions from the storage medium, the computer executes the method for ultrasonic imaging of any one of claims 1 to 12.

**100**

First determination module 110

Second determination module 120

Third determination module 130

Image generation module 140

**FIG. 1**

**200**

Determining a delay time of each pixel point in an effective region based on an echo signal of a focused wave  ⟍⟋210

↓

Determining reference pixel points from the effective region and delay times corresponding to the reference pixel points  ⟍⟋220

↓

Determining a delay time of each of one or more target pixel points in an artifact region based on the reference pixel points and the delay times corresponding to the reference pixel points  ⟍⟋230

↓

Generating an ultrasonic image based on the delay time of each pixel point in the effective region and the delay time of each of the one or more target pixel points in the artifact region  ⟍⟋240

**FIG. 2**

<u>**300**</u>

Determining three first areas based on position
information of each two of three reference pixel points
and position information of a target pixel point $\sim 310$

Determining a second area based on the position
information of the three reference pixel points $\sim 320$

Determining a delay time of the target pixel point based
on the three first areas, the second area, and delay times
of the three reference pixel points $\sim 330$

**FIG. 3**

**400**

Determining a first line segment and one or more second line segments based on position information of four reference pixel points and position information of a target pixel point ⟿410

Determining position information of one or more intermediate pixel points, wherein each of the one or more intermediate pixel points is an intersection point of the first line segments and one of the one or more second line segments ⟿420

Determining a delay time of each of the one or more intermediate pixel points based on the position information of the intermediate pixel point and position information and delay times of reference pixel points at two ends of a second line segment where the intermediate pixel point is located ⟿430

Determining a delay time of the target pixel point based on the delay times of the one or more intermediate pixel points ⟿440

**FIG. 4**

500

Determining first distances and a second distance based on position information of each of one or more intermediate pixel points and position information of reference pixel points at two ends of each of the one or more second line segments where the intermediate pixel point is located ⟍⟋510

For each of the first distances, ⟍⟋520-1

Determining a first distance ratio of the first distance to the second distance

⟍⟋520-2

Determining a first distance product result of a delay time of a reference pixel point corresponding to the first distance and the first distance ratio

⟍⟋530

Determining a delay time of the intermediate pixel point based on first distance product results corresponding to the first distances

**FIG. 5**

**600**

Determining third distances and a fourth distance based on position information of an intermediate pixel point and position information of a target pixel point
~610

For each of the third distances, ~620-1

Determining a second distance ratio of the third distance to the fourth distance

~620-2

Determining a second distance product result of a delay time of the intermediate pixel point corresponding to the third distance and the second distance ratio corresponding to the third distance

~630

Determining a delay time of the target pixel point based on second distance product results corresponding to the third distances

**FIG. 6**

**FIG. 7A**

**FIG. 7B**

**FIG. 7C**

**FIG. 8A**

**FIG. 8B**

**FIG. 8C**

**FIG. 9A**

**FIG. 9B**

## INTERNATIONAL SEARCH REPORT

| International application No. |
| --- |
| **PCT/CN2023/135521** |

**A.    CLASSIFICATION OF SUBJECT MATTER**

G01S15/89(2006.01)i;  A61B8/00(2006.01)i

According to International Patent Classification (IPC) or to both national classification and IPC

**B.    FIELDS SEARCHED**

Minimum documentation searched (classification system followed by classification symbols)

IPC:  G01S A61B

Documentation searched other than minimum documentation to the extent that such documents are included in the fields searched

Electronic data base consulted during the international search (name of data base and, where practicable, search terms used)

CNTXT; ENTXT; CNKI; VEN; ENTXT; ENTXTC; IEEE: 联影医疗, 张建敏, 史爱伟, 点源, 区间, 区域, 伪影, 像素, 延迟, 延时, 角度, 距离, 面积, 参考, ultrasonic, delay, area, pixel point, distance, angle

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 115712120 A (WUHAN UNITED IMAGING HEALTHCARE CO., LTD.) 24 February 2023 (2023-02-24) <br> description, paragraphs [0005]-[0242], and figures 1-19 | 1-3, 6-23 |
| X | US 2016174938 A1 (HITACHI ALOKA MEDICAL LTD.) 23 June 2016 (2016-06-23) <br> description, paragraphs [0005]-[0104], and figures 1-17 | 1-7, 13-16, 22, 23 |
| A | CN 105997137 A (KONICA MINOLTA INC.) 12 October 2016 (2016-10-12) <br> entire document | 1-23 |
| A | CN 110622034 A (KONINKL PHILIPS NV) 27 December 2019 (2019-12-27) <br> entire document | 1-23 |
| A | CN 110840484 A (SONOSCAPE MEDICAL CORP.) 28 February 2020 (2020-02-28) <br> entire document | 1-23 |

☐ Further documents are listed in the continuation of Box C.    ☑ See patent family annex.

| | |
| --- | --- |
| *    Special categories of cited documents: <br> "A"    document defining the general state of the art which is not considered to be of particular relevance <br> "D"    document cited by the applicant in the international application <br> "E"    earlier application or patent but published on or after the international filing date <br> "L"    document which may throw doubts on priority claim(s) or which is cited to establish the publication date of another citation or other special reason (as specified) <br> "O"    document referring to an oral disclosure, use, exhibition or other means <br> "P"    document published prior to the international filing date but later than the priority date claimed | "T"    later document published after the international filing date or priority date and not in conflict with the application but cited to understand the principle or theory underlying the invention <br> "X"    document of particular relevance; the claimed invention cannot be considered novel or cannot be considered to involve an inventive step when the document is taken alone <br> "Y"    document of particular relevance; the claimed invention cannot be considered to involve an inventive step when the document is combined with one or more other such documents, such combination being obvious to a person skilled in the art <br> "&"    document member of the same patent family |

| Date of the actual completion of the international search | Date of mailing of the international search report |
| --- | --- |
| **22 February 2024** | **08 March 2024** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/135521**

| Patent document cited in search report | | | Publication date (day/month/year) | Patent family member(s) | | | Publication date (day/month/year) |
|---|---|---|---|---|---|---|---|
| CN | 115712120 | A | 24 February 2023 | None | | | |
| US | 2016174938 | A1 | 23 June 2016 | US | 11160533 | B2 | 02 November 2021 |
| CN | 105997137 | A | 12 October 2016 | None | | | |
| CN | 110622034 | A | 27 December 2019 | None | | | |
| CN | 110840484 | A | 28 February 2020 | None | | | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211513999

- CN 103837608 B